# EUROPÄISCHE PATENTSCHRIFT

(11) **EP 0 371 414 B1**
(45) Veröffentlichungstag und Bekanntmachung des Hinweises auf die Patenterteilung: **09.07.1997**
(21) Anmeldenummer: 89121788.7
(22) Anmeldetag: 25.11.1989
(51) Int. Cl.: C07H 15/04, A61K 31/70

(54) **Glykosphingolipide mit kopplungsfähiger Gruppe im Sphingoidanteil, ihre Herstellung und Verwendung**
Glycosphingolipids with a coupling group in the sphingolipid part, their preparation and use
Glycosphingolipides avec un groupe couplant dans la partie sphingolipide, leur préparation et utilisation

(30) Priorität: 27.11.1988 DE 3840044
(43) Veröffentlichungstag der Anmeldung: 06.06.1990
(73) Patentinhaber: BEHRINGWERKE Aktiengesellschaft, 35001 Marburg (DE)
(72) Erfinder: Wiegand, Herbert, Prof.Dr., D-3550 Marburg (DE); Bosslet, Silke, D-3550 Marburg (DE)

(56) Entgegenhaltungen:
- FR-A- 2 403 098
- CHEMICAL ABSTRACTS, Band 81, Nr. 23, 9. Dezember 1974, Seite 364, Zusammenfassung Nr. 149983c, Columbus, Ohio, US; R.A. LAINE et al.: "Glycosphingolipids covalently linked to agarose gel or glass beads. Use of the compounds for purification of antibodies directed against globoside and
- hematoside", & J. BIOL. CHEM. 1974, 249(14), 4460-6
- CHEMICAL ABSTRACTS, Band 85, Nr. 13, 27. September 1976, Seite 452, Zusammenfassung Nr. 92009r, Columbus, Ohio, US; K. UEMURA et al.: "Chemical and hemolytic properties of sphingolipids modified by ozonolysis and reduction", & J. BIOCHEM. (TOKYO) 1976, 79(6), 1253-61
- CHEMICAL ABSTRACTS, Band 86, Nr. 5, 31. Januar 1977, Seite 130, Zusammenfassung Nr. 27041b, Columbus, Ohio, US; H. WIEGANDT et al.: "Studies of the ligand binding to cholera toxin. I. The lipophilic moiety of sialoglycolipids", & HOPPE-SEYLER'S Z. PHYSIOL. CHEM. 1976, 357(11), 1637-46
- CHEMICAL ABSTRACTS, Band 97, Nr. 21, 22. November 1982, Seite 343, Zusammenfassung Nr. 177310e, Columbus, Ohio, US; B. VENERANDO et al.: "Interactions of ganglioside Gm1 with human and fetal calf serums. Formation of ganglioside-serum albumin complexes", & BIOCHIM. BIOPHYS. ACTA 1982, 692(1), 18-26
- CHEMICAL ABSTRACTS, Band 82, Nr. 19, 12. Mai 1975, Seite 384, Zusammenfassung Nr. 123050s, Columbus, Ohio, US; R. ARNON et al.: "Lipid-specific antibodies
- elicited with synthetic lipid conjugates", & CHEM. PHYS. LIPIDS 1974, 13(4), 352-66
- CHEMICAL ABSTRACTS, Band 93, Nr. 11, 15. September 1980, Seite 366, Zusammenfassung Nr. 110189w, Columbus, Ohio, US; D.L. MacDONALD et al.: "Notes on improved procedures for the chemical modification and degradation of glycosphingolipids", & J. LIPID RES. 1980, 21(5), 642-5
- A.J.CUMBER et al. Methods in Enzymology Vol.112, p.207-224 (1985)

## Beschreibung

Die Erfindung betrifft die chemische Modifizierung der Sphingoid-Anteile von Glykosphingolipiden. Durch eine Reihe von Reaktionen konnte in der Position der Kohlenstoff-Doppelbindung im Sphingoid-Anteil nach Abspaltung des langkettigen Aldehyds eine Aminogruppe eingeführt werden.

Glykosphingolipide (Formel I) sind Plasmamembranlipide, die aus einem hydrophilen Kohlenhydrat-Anteil und einem hydrophoben Ceramid-Anteil bestehen. Der Ceramid-Anteil setzt sich aus Sphingosin, einem langkettigen Aminoalkohol und einer amidartig gebundenen Fettsäure zusammen.

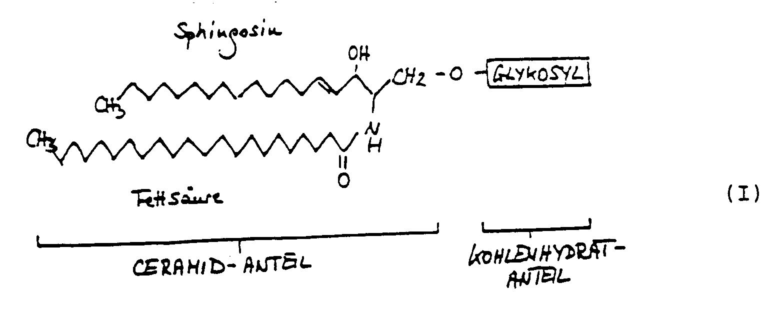

Mit diesem zweischwänzigen hydrophoben Anteil sind Glykosphingolipide so in der äußeren Plasmamembran verankert, daß ihre Oligosaccharidketten in den Extrazellulärraum ragen.

Trotz intensiver Forschungsarbeit ist die biologische Funktion von Glykosphingolipiden noch nicht genau bekannt; sie scheinen jedoch eine Rolle bei der Regulation des Zellwachstums- und Differenzierungsverhaltens zu spielen. Befunde, die zeigen, daß vor allem sialinsäurehaltige Glykosphingolipide, Ganglioside, auf einigen Tumoren neuroektodermalen Ursprungs in relativ großer Menge vorkommen, während sie auf Normalgewebe in geringeren Mengen exprimiert werden, haben sie als tumor-assoziierte Antigene für Tumor-Diagnose und Tumor-Therapie interessant werden lassen.

Das besondere Interesse gilt hierbei dem Kohlenhydrat-Anteil. Es konnte bereits gezeigt werden (deutsche Patentanmeldung P 38 37 623.7) daß entsprechende Sialylzucker, aus einer Quelle wie Kuh-Kolostrum isoliert und auf ein Trägerprotein gekoppelt, Epitope von Gangliosiden imitieren können: monoklonale Antigangliosid-Antikörper reagieren mit diesen Neoglykoproteinen.

Um eine universellere, für alle Glykosphingolipide anwendbare gezielte Kopplung etablieren zu können, ist die chemische Modifizierung des Sphingoid-Anteils von Glykosphingolipiden für weitere Arbeiten auf diesem Gebiet notwendig. Dies betrifft die Darstellung von synthetischen Glykosphingolipid-Vakzinen. Zusätzlich ist die Einführung einer kopplungsfähigen Gruppe auch für Fragestellungen in der Grundlagenforschung relevant, z.B. können an Reporterenzyme gekoppelte Glykosphingolipide bei histochemischen Untersuchungen eingesetzt werden, etwa zur Charakterisierung von Säugerlektinen als Rezeptoren für Glykosphingolipide.

Die Erfindung zeigt, daß durch die unten geschilderten Reaktionen eine Amino-Gruppe als funktionelle Gruppe in den Sphingoid-Anteil von Glykosphingolipiden nach Abspaltung eines langkettigen Aldehyds eingeführt werden kann. Dabei bleibt der Kohlenhydrat-Anteil unverändert glykosidisch am Ceramid-Anteil gebunden.

Die eingeführte Amino-Gruppe ermöglicht eine Reihe weiterer Reaktionen, z.B. die Kopplung an heterobifunktionelle Reagenzien zur Synthese von Glykosphingolipid-Konjugaten und deren Einsatz als synthetische Vakzine in der Therapie von Tumoren neuroektodermalen Ursprungs.

Neben der Einführung einer Aminogruppe besteht auch die Möglichkeit, das Zwischenprodukt (es trägt eine Aldehyd-Gruppe) direkt mit anderen, kopplungsfähige Gruppen tragenden Molekülen reagieren zu lassen, z.B. Amino-Gruplen von Proteinen, Kopplungsreagenzien etc. Bei den Reaktionen ist zu bedenken, daß das Zwischenprodukt mit der Aldehyd-Gruppe nicht sehr stabil ist und im Alkalischen der Kohlenhydrat-Anteil eliminiert wird.

Die prinzipiellen Reaktionen zur Einführung der kopplungsfähigen Gruppe an der Position der Kohlenstoffdoppelbindung im Sphingoidanteil sind wie folgt:
1. Die o.g. Doppelbindung wird durch Ozon gespalten (H. Wiegandt und G. Baschang, Z. Naturforschung 206, (1965), 164-166) und die in Methanol als Zwischenprodukte entstehenden Methoxyhydroperoxid-Derivate (H. Wiegandt, Ang. Chem. Intl. Ed. 7, (1968) 87-96) durch Zugabe von Dimethylsulfid zum Aldehyd reduziert (Pappas et al., Tetrahedron Letters, 36 (1966), 4273-4278). 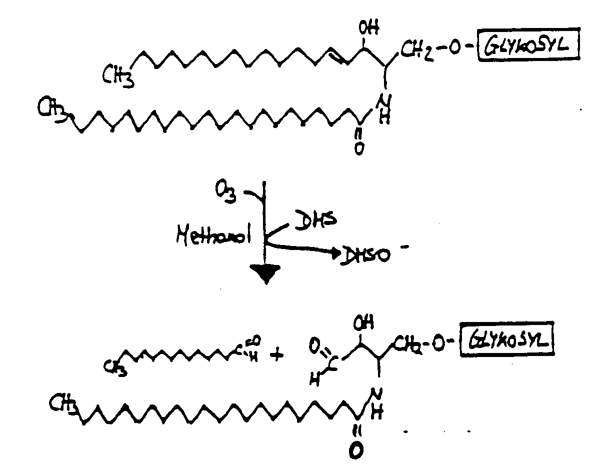 Die entstandene Verbindung ist nur im Neutralen und im Sauren stabil; im Alkalischen wird der Kohlenhydrat-Anteil eliminiert.
2. Nach Entfernung des langkettigen Aldehyds durch Ausschütteln in Hexan wird im weiteren das Ozonolyseprodukt in Methanol unter Zusatz von 1 M Ammoniumacetat und Natriumcyanoborhydrid reduktaminiert (Wiegandt und Ziegler, Hoppe-Seyler's Physiol. Chem. 355, (1974), 11-18).

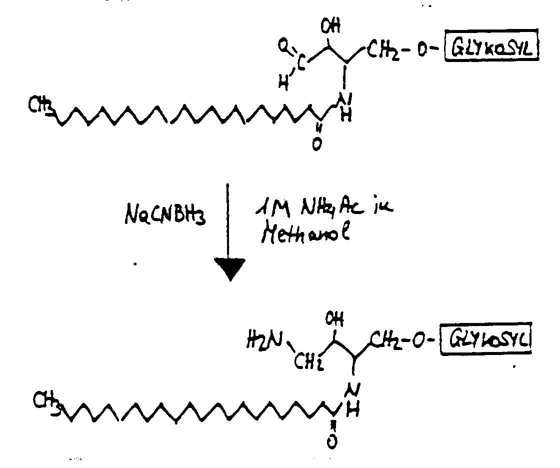

Alle Reaktionsschritte verlaufen mit einer sehr hohen Ausbeute und können dünnschichtchromatographisch verfolgt werden: Laufmittel ist Chloroform/Methanol/Wasser (65:25:4). Der Nachweis der Reaktionsprodukte auf den Dünnschichtchromatographie-Platten erfolgt mit Jod-Dampf, Ninhydrin- bzw. Fluoram- und Orcin-Sprühreagenz.

Das reduktaminierte Ozonolyseprodukt ist auf der Dünnschichtchromatographie-Platte Ninhydrin- und Fluoram-positiv und kann mit Aminogruppen-spezifischen Reagenzien wie Fluordinitrobenzol (Sanger's Reagenz), (Itasaka und Hori, J. Biochem. 85, (1979), 1469-1481) oder SPDP (N-Succinimidyl-3-(2-pyridyldithio)propionat) (Carlsson et al., Biochem. J. 173, (1978), 723-737) vollständig umgesetzt werden.

Die Erfindung betrifft folglich Glykosphingolipide mit kopplungsfähiger Gruppe in der Position der Kohlenstoffdoppelbindung, wobei ein langkettiger Aldehyd abgespalten wird, das Molekül dabei ansonsten intakt und insbesondere die Glykosidbindung des Zuckeranteils erhalten bleibt und die kopplungsfähige Gruppe eine Aminogruppe oder eine Aldehydgruppe ist, sowie Verfahren zu ihrer Herstellung und ihre Verwendung zur Kopplung an geeignete Reaktionspartner und als Bestandteil von Arzneimitteln.

Die Erfindung ist ferner in dem Beispiel und den Patentansprüchen offenbart.

### Beispiel A:

### 1. Ozonolyse

In einem typischen Reaktionsansatz wurden 6 mg Cerebrosid (Galaktosylceramid) in 5 ml Methanol gelöst und der Ansatz bei Raumtemperatur ozonolysiert (1 Blase/sec), bis unverbrauchtes Ozon durch die Violettfärbung der KJ-Stärke-Nachweislösung angezeigt wurde.

### 2. Reduktion zum Aldehyd

Anschließend wurde der Ansatz mit Stickstoff begast, 30 µl Dimethylsulfid zugegeben und über Nacht stehengelassen. Nach kaltem Einrotieren der Lösung am Rotationsverdampfer wurde der bei der Ozonolyse freigewordene langkettige Aldehyd durch Ausschütteln in Hexan (3 x 2 ml) entfernt.

### 3. Reduktaminierung des Ozonolyseproduktes

Der Extraktionsrückstand wurde in 4 ml 1 M Ammoniumacetat in Methanol gelöst, 15 mg Natriumcyanoborhydrid dazugegeben und der Ansatz bei 80°C 4-5 Stunden unter Rückfluß gekocht.

Das reduktaminierte Ozonolyseprodukt wurde anschließend über Reversed-Phase (RP18)-Chromatographie und Kieselgelsäulchen entsalzt und gereinigt.

### 4. Umsetzung des reduktaminierten Ozonolyseprodukts mit Fluordinitrobenzol

Ein Aliquot des reduktaminierten Ozonolyseproduktes wurde in 500 µl Methanol gelöst und dem Ansatz 4 Tropfen Triethylamin und 20 µl 5% Fluordinitrobenzol in Ethanol zugegeben. Die Umsetzung erfolgte bei Raumtemperatur bei gelegentlichem Schütteln in 1-2 Stunden.

Das entstandene Dinitrophenyl-Derivat konnte sofort aufgrund seiner Gelbfärbung auf der Dünnschichtchromatographie-Platte identifiziert werden (HPTLC-Platte, Kieselgel 60 (Merck, Darmstadt); Laufmittel: Chloroform/Methanol/Wasser, 65:25:4)

### Beispiel B:

Kopplung von reduktaminierten Ozonolyseprodukten der Ganglioside GM3, GD3, GM2 und GM1 an Humanes Serum Albumin (HSA) mittels des heterobifunktionellen Kopplungsreagenzes N-Succinimidyl-3-(2-pyridyldithio)propionat (SPDP); Synthese von Konjugaten mit einem Derivatisierungsrgrad von 16 - 18 Gangliosid-Derivaten pro HSA-Molekül

### 1. Darstellung der reduktaminierten Ozonolyseprodukte der Ganglioside GM3, GD3, GM2 und GM1

Die Darstellung erfolgte wie im Beispiel A für Cerebrosid unter 1.-3.- beschrieben.
Massenspektrum-Analysen der GM1- und GM3-Derivate bestätigten der erwartete Struktur.

Die weiteren Reaktionsschritte:

### 2. Umsetzung der reduktaminierten Ozonolyseprodukte mit SPDP

### 3. Umsetzung von HSA mit SPDP

### 4. Reduktion des HSA-SPDP-Derivates

### 5. Kopplung des Gangliosid- mit dem Protein-Derivat

sowie die entsprechenden Nachweismethoden entsprechen weitgehend der Methode von J. Carlsson et al. (1987) Biochem. J. 173, 723-737 und wie in der Patentanmeldung DE P 38 37 623.7 vorgeschlagen. Die Schritte 2. und 3. wurden in 0.1 M Natriumphosphat-Puffer, pH 7.5 mit 3 bis 5fachem molaren (bei 3. bezogen auf freie epsilon-Aminolysylgruppen) SPDP-Überschuß durchgeführt. Die Abtrennung des Protein-SPDP-Derivates von 3. erfolgte über eine Sephadex G-25-Säule, die mit dem Puffer für die nachfolgenden Reaktionen (0.1 M Natriumphosphatpuffer, pH 6,5 mM EDTA) eluiert wurde. Die Reinigung der mit SPDP umgesetzten Gangliosid-Derivate erfolgte über Reversed-Phase (RP18)-Chromatographie. Die dünnschichtchromatografische Identifizierung der einzelnen Zwischenprodukte erfolgte aufgrund des veränderten Laufverhaltens auf Kieselgel G-60 Platten in den Laufmitteln Chloroform/Methanol/0.2% wässriges Kalziumchlorid (65:25:4) oder (50:40:10).

Schritt 4. wurde wie folgt durchgeführt:

Unter Zusatz von 25 mM Dithiothreitol wurden die durch die Derivatisierung neu eingeführten Disulfidbrücken des HSA-SPDP-Derivates in 0.1 M Natriumphosphat-Puffer, pH 6,5 mM EDTA unter Abspaltung von 2-Thiopyridon reduziert. Unter diesen Reaktionsbedingungen werden die nativen Disulfidbrücken des Proteins nicht reduziert. Die Reaktion erfolgte bei Raumtemperatur und die Reaktionszeit betrug 1-2 Stunden.

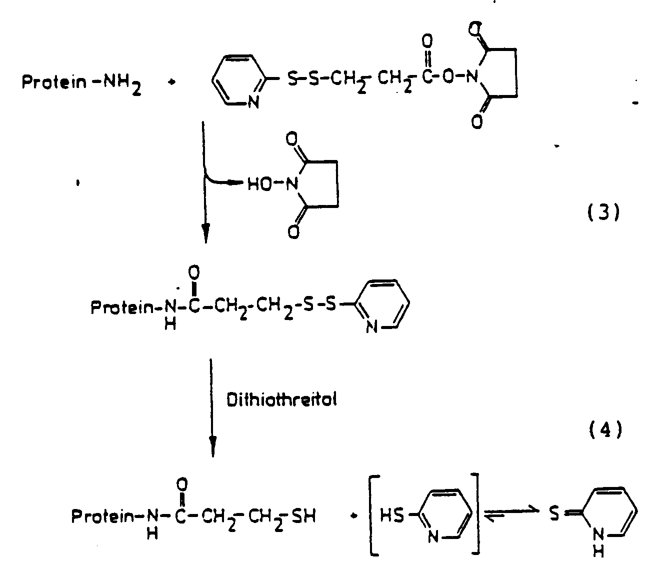

Die Abtrennung des reduzierten HSA-SPDP-Derivates erfolgte über eine Sephadex G-25 Säule mit 0.1 M Natriumphosphat-Puffer, pH 6, 5 mM EDTA als Elutionspuffer.

Durch Umsetzung von HSA mit SPDP-Unterschuß konnten gezielt HSA-Derivate mit gewünschtem Derivatisierungsgrad hergestellt werden.

Die Gangliosid-Derivate wurden mit HSA umgesetzt, das mit 16-18 SPDP-Molekülen derivatisiert war. Die Umsetzung erfolgte vollständig; der Derivatisierungsgrad des Kopplungsproduktes betrug 16 - 18 Gangliosid-Derivate (jeweils der Ganglioside GM3, GD3, GM2 und GM1) pro HSA-Molekül.

Die Kopplung (Reaktionsschritt 5.) erfolgte im einzelnen wie folgt:

Reduziertes HSA-SPDP-Derivat wurde sofort mit dem Gangliosid-SPDP-Derivat umgesetzt. Das Gangliosid-SPDP-Derivat wurde in 1-5fachem molaren Überschuß, bezogen auf epsilon-Aminolysylgruppen des Proteins, eingesetzt, die Reaktionszeit betrug 24-48 Stunden bei Raumtemperatur.

## Patentansprüche (Patentansprüche für folgende(n) Vertragsstaat(en): AT, BE, CH, LI, DE, FR, GB, IT, LU, NL, SE)

1. Glykosphingolipide der Formeln (2) oder (3)

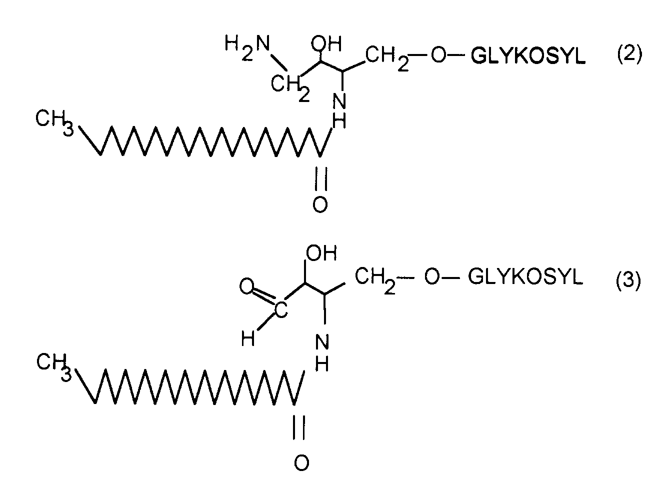

2. Verfahren zur Herstellung von Verbindungen nach Anspruch 1, dadurch gekennzeichnet, daß ausgehend von Glykosphingolipiden, welche keine Schutzgruppen tragen, eine Ozonolysereaktion durchgeführt und das als Zwischenprodukt entstehende Methoxyhydroperoxid-Derivat zum Aldehyd reduziert wird.

3. Verfahren zur Herstellung von Verbindungen nach Anspruch 1, dadurch gekennzeichnet, daß ausgehend von Glykosphingolipiden nach reduktiver Ozonolyse eine Reduktaminierung mit einem Alkalicyanoborhydrid durchgeführt wird.

4. Kopplungsverbindungen, die durch Kopplung mit Verbindungen nach Anspruch 1 erhalten werden.

5. Verbindungen nach Anspruch 1, dadurch gekennzeichnet, daß heterobifunktionelle Reagenzien angekoppelt sind.

6. Kopplungsverbindungen nach Anspruch 4 oder 5, dadurch gekennzeichnet, daß Proteine angekoppelt sind.

7. Verwendung von Verbindungen nach Anspruch 1 zur Synthese von Kopplungsverbindungen.

8. Arzneimittel, die Verbindungen nach Anspruch 1, 4, 5 oder 6 enthalten.

## Patentansprüche (Patentansprüche für folgende(n) Vertragsstaat(en): ES)

1. Verfahren zur Herstellung von Glykosphingolipiden der Formel (2) oder (3) 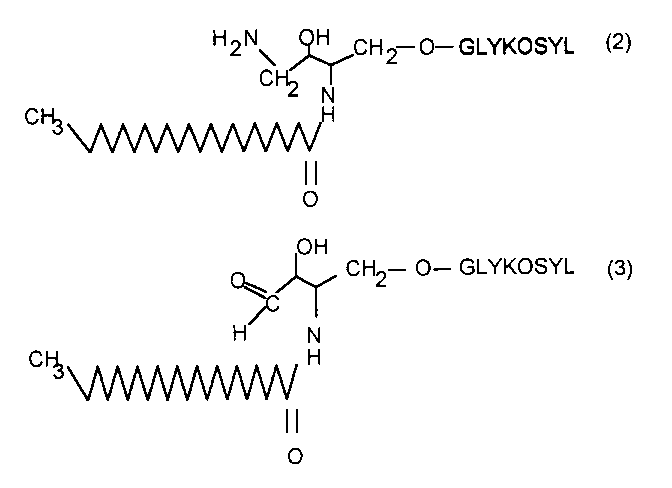 dadurch gekennzeichnet, daß ausgehend von Glykosphingolipiden, welche keine Schutzgruppen tragen, eine Ozonolysereaktion durchgeführt und das als Zwischenprodukt entstehende Methoxyhydroperoxid-Derivat zum Aldehyd reduziert wird.

2. Verfahren zur Herstellung von Verbindungen nach Anspruch 1, dadurch gekennzeichnet, daß ausgehend von Glykosphingolipiden nach reduktiver Ozonolyse eine Reduktaminierung mit einem Alkalicyanoborhydrid durchgeführt wird.

3. Verfahren zur Herstellung von Kopplungsverbindungen, dadurch gekennzeichnet, daß geeignete Moleküle und Verbindungen der Formel (2) oder (3) umgesetzt werden.

4. Verfahren zur Herstellung von reaktiven Kopplungsverbindungen, dadurch gekennzeichnet, daß heterobifunktionelle Reagenzien an Verbindungen der Formel (2) oder (3) angekoppelt werden.

5. Verfahren zur Herstellung von Proteinkonjugaten, dadurch gekennzeichnet, daß Verbindungen der Formel (2) oder (3) oder nach Anspruch 4 hergestellte Verbindungen mit Protein umgesetzt werden.

6. Verfahren zur Herstellung von Arzneimitteln, dadurch gekennzeichnet, daß nach Anspruch 1, 2, 3, 4 oder 5 hergestellte Verbindungen mit geeigneten Trägermaterialien versetzt werden.

## Claims (Claims for the following Contracting State(s): AT, BE, CH, LI, DE, FR, GB, IT, LU, NL, SE)

1. A glycosphingolipid of formulae (2) or (3)

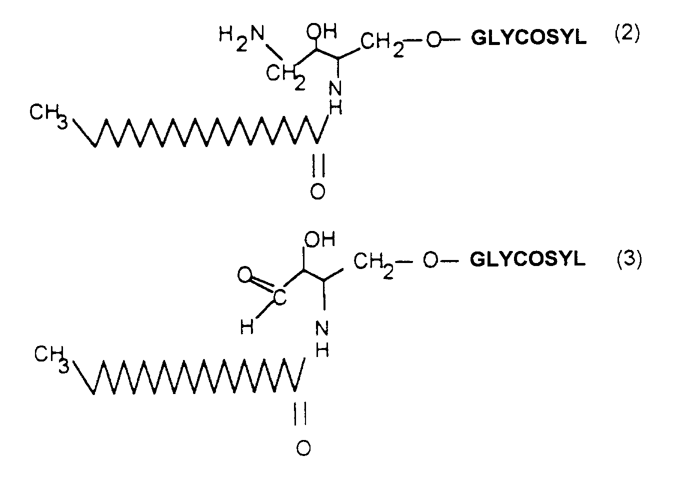

2. A process for preparing compounds as claimed in claim 1, which comprises starting from glycosphingolipids without protective groups, carrying out an ozonolysis reaction, and reducing the methoxy hydroperoxide derivative produced as intermediate to the aldehyde.

3. A process for preparing compounds as claimed in claim 1, wherein reductive ozonolysis of the starting glycosphingolipids is followed by reductive amination with an alkali metal cyanoborohydride.

4. A coupling compound obtained by coupling with compounds as claimed in claim 1.

5. A compound as claimed in claim 1, wherein heterobifunctional reagents are coupled on.

6. A coupling compound as claimed in claim 4 or 5, wherein proteins are coupled on.

7. The use of compounds as claimed in claim 1 for synthesizing coupling compounds.

8. A pharmaceutical comprising compounds as claimed in claim 1, 4, 5 or 6.

## Claims (Claims for the following Contracting State(s): ES)

1. A process for preparing glycosphingolipids of the formula (2) or (3) 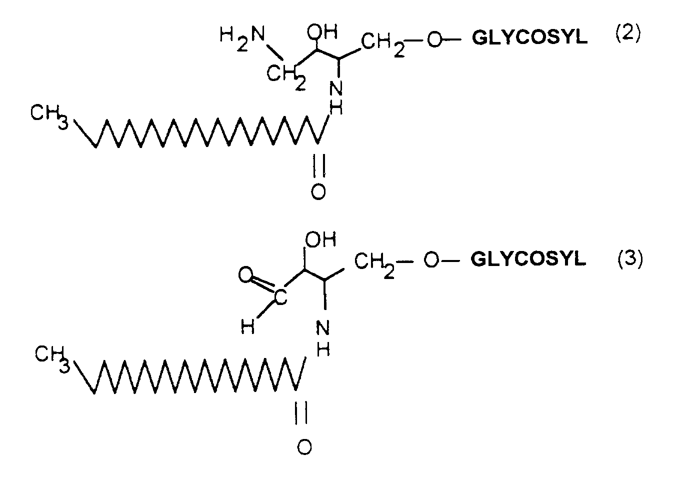 which comprises starting from glycosphingolipids without protective groups, carrying out an ozonolysis reaction, and reducing the methoxy hydroperoxide derivative produced as intermediate to the aldehyde.

2. A process for preparing compounds as claimed in claim 1, wherein reductive ozonolysis of the starting glycosphingolipids is followed by reductive amination with an alkali metal cyanoborohydride.

3. A process for preparing coupling compounds, which comprises reacting suitable molecules and compounds of the formula (2) or (3).

4. A process for preparing reactive coupling compounds, which comprises coupling heterobifunctional reagents onto compounds of the formula (2) or (3).

5. A process for preparing protein conjugates, which comprises reacting compounds of the formula (2) or (3) or compounds prepared as claimed in claim 4 with protein.

6. A process for producing pharmaceuticals, which comprises mixing compounds prepared as claimed in claim 1, 2, 3, 4 or 5 with suitable carrier materials.

## Revendications (Revendications pour l'(les) Etat(s) contractant(s) suivant(s): AT, BE, CH, LI, DE, FR, GB, IT, LU, NL, SE)

1. Glycosphingolipides de formule (2) ou (3)

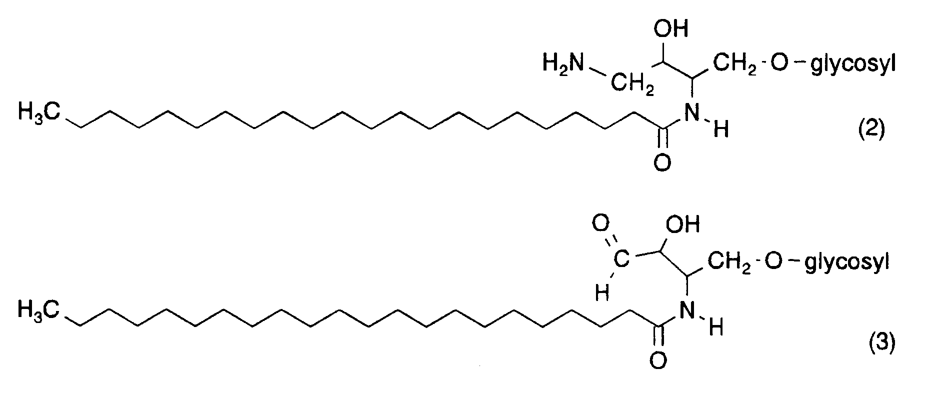

2. Procédé pour la préparation de composés selon la revendication 1, caractérisé en ce que l'on conduit une réaction d'ozonolyse à partir de glycosphingolipides qui ne portent pas de groupes protecteurs, et que le dérivé méthoxyhydroperoxyde obtenu comme produit intermédiaire est réduit en aldéhyde.

3. Procédé pour la préparation de composés selon la revendication 1, caractérisé en ce que l'on conduit une amination réductrice avec un cyanoborohydrure de métal alcalin à partir de glycosphingolipides, après ozonolyse réductrice.

4. Composés de couplage obtenus par couplage avec des composés selon la revendication 1.

5. Composés selon la revendication 1, caractérisés en ce que des réactifs hétérobifonctionnels sont couplés.

6. Composés de couplage selon la revendication 4 ou 5, caractérisés en ce que des protéines sont couplées.

7. Utilisation de composés selon la revendication 1 pour la synthèse de composés de couplage.

8. Médicaments qui contiennent des composés selon la revendication 1, 4, 5 ou 6.

## Revendications (Revendications pour l'(les) Etat(s) contractant(s) suivant(s): ES)

1. Procédé pour la préparation de glycosphingolipides de formule (2) ou (3) 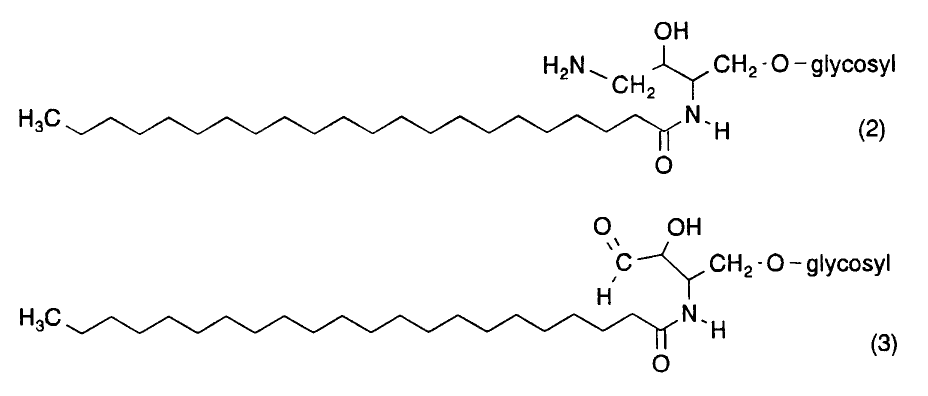 caractérisé en ce que, à partir de glycosphingolipides qui ne portent pas de groupes protecteurs, on conduit une réaction d'ozonolyse et que l'on réduit le dérivé méthoxyhydroperoxyde obtenu comme produit intermédiaire en aldéhyde.

2. Procédé pour la préparation de composés selon la revendication 1, caractérisé en ce que l'on conduit une amination réductrice avec un cyanoborohydrure de métal alcalin à partir de glycosphingolipides, après ozonolyse réductrice.

3. Procédé pour la préparation de composés de couplage, caractérisé en ce que l'on fait réagir des molécules appropriés et des composés de formule (2) ou (3).

4. Procédé pour la préparation de composés de couplage réactifs, caractérisé en ce que des réactifs hétérobifonctionnels sont couplés à des composés de formule (2) ou (3).

5. Procédé pour la préparation de conjugués de protéine, caractérisé en ce que l'on fait réagir des composés de formule (2) ou (3) ou des composés préparés selon la revendication 4 avec une protéine.

6. Procédé pour la préparation de médicaments, caractérisé en ce que l'on mélange des composés préparés selon la revendication 1, 2, 3, 4 ou 5 avec des véhicules appropriés.
